# EUROPEAN PATENT APPLICATION

(11) **EP 3 943 589 A1**
(43) Date of publication of application: **26.01.2022**
(21) Application number: 20916238.7
(22) Date of filing: 18.06.2020
(51) Int. Cl.: C12M 1/00, C12Q 1/68

(54) **NUCLEIC ACID TEST KIT**

(30) Priority: 10.06.2020 CN 202010522831
(71) Applicant: Beijing Baicare Biotechnology Co., Ltd., Beijing 100089 (CN)
(72) Inventor: WANG, Hu, Beijing 100089 (CN); LI, Yongfeng, Beijing 100089 (CN); ZHANG, Xinjian, Beijing 100089 (CN); HU, Zaibing, Beijing 100089 (CN); ZHANG, Guohao, Beijing 100089 (CN)
(74) Representative: Petraz, Gilberto Luigi
(86) International application number: PCT/CN2020/096723
(87) International publication number: WO 2021/248531

(57) **Abstract**

The invention provides a nucleic acid testing cassette, including a substrate, a liquid storage component, a solid-reagent storage component, and an amplification reaction region, wherein the substrate is connected to the amplification reaction region; the liquid storage component and the solid-reagent storage component are disposed on the substrate, respectively; the liquid storage component is communicated with the solid-reagent storage component through a micro flow channel; and the solid-reagent storage component is communicated with the amplification reaction region through a micro flow channel. The invention has the following beneficial effects: all the reagents required for nucleic acid extraction and amplification can be internally disposed on an integrated cassette, a liquid reagent is stored in the liquid storage component, and a dry-powder reagent is stored in the solid-reagent storage component, so that a user only needs to add a sample; therefore, the nucleic acid testing cassette is extremely easy to operate and truly suitable for primary users to use.

## Description

### BACKGROUND

### Technical Field

The invention relates to a nucleic acid testing kit, and in particular, relates to a nucleic acid testing cassette.

### Description of Related Art

Current nucleic acid testing kits are low in integration level and complex to use. Moreover, it is necessary for users to add some reagents manually, which often leads to mis-operations. As a result, testing results are inaccurate, and these nucleic acid testing kits are not suitable for primary users to use.

### SUMMARY

To solve the problems in the prior art, the invention provides a nucleic acid testing cassette.

The invention provides a nucleic acid testing cassette, including a substrate, a liquid storage component, a solid-reagent storage component, and an amplification reaction region, wherein the substrate is connected to the amplification reaction region; the liquid storage component and the solid-reagent storage component are disposed on the substrate, respectively; the liquid storage component is communicated with the solid-reagent storage component through a micro flow channel; and the solid-reagent storage component is communicated with the amplification reaction region through a micro flow channel.

As a further improvement of the invention, the liquid storage component includes a sample storage component, an extraction-reagent storage component, and a waste-liquid immobilization cavity; the substrate is provided with a nucleic acid immobilization-extraction reaction pool; an output end of the sample storage component and an output end of the extraction-reagent storage component are communicated with an input end of the nucleic acid immobilization-extraction reaction pool through a micro flow channel, respectively; and an output end of the nucleic acid immobilization-extraction reaction pool is communicated with the solid-reagent storage component and the waste-liquid immobilization cavity through a micro flow channel, respectively.

As a further improvement of the invention, the extraction-reagent storage component includes a plurality of separate chambers, each of which includes a tube wall, a plunger, and a diaphragm; the plungers are disposed above the tube walls; the diaphragms are disposed below the tube walls; the tube walls, the plungers, and the diaphragms enclose a liquid-reagent storage cavity; the substrate is provided with sharp protrusions in one-to-one correspondence to the chambers; and the sharp protrusions are disposed below the diaphragms.

As a further improvement of the invention, the chambers at least include a first chamber storing a binding solution, a second chamber storing a rinsing solution, and a third chamber storing an eluent; the first chamber is communicated with the sample storage component through a micro flow channel; a micro flow channel between the sample storage component and the nucleic acid immobilization-extraction reaction pool is provided with a first fluid isolating valve; a micro flow channel between the second chamber and the nucleic acid immobilization-extraction reaction pool is provided with a second fluid isolating valve; a micro flow channel between the third chamber and the nucleic acid immobilization-extraction reaction pool is provided with a third fluid isolating valve; a micro flow channel between the nucleic acid immobilization-extraction reaction pool and the solid-reagent storage component is provided with a fourth fluid isolating valve; a micro flow channel between the nucleic acid immobilization-extraction reaction pool and the waste-liquid immobilization cavity is provided with a fifth fluid isolating valve; and a micro flow channel between the solid-reagent storage component and the amplification reaction region is provided with a sixth fluid isolating valve.

As a further improvement of the invention, the chambers further include a preamplification reagent chamber storing a preamplification reagent; and the preamplification reagent chamber is communicated with the nucleic acid immobilization-extraction reaction pool through a micro flow channel, on which a seventh fluid isolating valve is disposed.

As a further improvement of the invention, the substrate is provided with a plurality of grooves that are communicated with each other; a cover piece is attached to the substrate, and encloses the grooves to form micro flow channels; and the substrate is provided with a plurality valve seat structures, on which the first fluid isolating valve, the second fluid isolating valve, the third fluid isolating valve, the fourth fluid isolating valve, the fifth fluid isolating valve, and the sixth fluid isolating valve are installed, respectively.

As a further improvement of the invention, the waste-liquid immobilization cavity is provided with first and second air passage interfaces that may be independently controlled to be opened or closed; the sample storage component is communicated with the first air passage interface; and the third chamber or the preamplification reagent chamber is communicated with the second air passage interface.

As a further improvement of the invention, the sample storage component is internally provided with a first magnetic rotor; the nucleic acid immobilization-extraction reaction pool is internally provided with a second magnetic rotor; a first magnet rotating mechanism for driving the first magnetic rotor to rotate is installed on the sample storage component; a second magnet rotating mechanism for driving the second magnetic rotor to rotate is installed on the nucleic acid immobilization-extraction reaction pool; a first heating module is installed on the nucleic acid immobilization-extraction reaction pool; and a second heating module is installed on the amplification reaction region.

As a further improvement of the invention, the sample storage component mainly consists of a cavity and a cover; the cavity of the sample storage component internally pre-stores nucleic-acid-capturing magnetic beads required for use in a nucleic acid extraction process.

As a further improvement of the invention, a porous water-absorbing material is contained in the waste-liquid immobilization cavity.

As a further improvement of the invention, the amplification reaction region mainly consists of a plurality of reaction wells and pipes connecting the reaction wells; the amplification reaction region is connected to the substrate through a connecting piece; and a fluorescence-imaging processing module is installed above the amplification reaction region.

The invention has the following beneficial effects: with the technical solutions described above, all the reagents required for nucleic acid extraction and amplification can be internally disposed on an integrated cassette, a liquid reagent is stored in the liquid storage component, and a dry-powder reagent is stored in the solid-reagent storage component, so that a user only needs to add a sample; therefore, the nucleic acid testing cassette is extremely easy to operate and truly suitable for primary users to use.

### BRIEF DESCRIPTION OF THE DRAWINGS

FIG. 1 is an overall schematic diagram of a nucleic acid testing cassette according to the invention.
FIG. 2 is a schematic diagram of a nucleic acid testing cassette according to the invention.
FIG. 3 is a schematic structural diagram of chambers of a nucleic acid testing cassette according to the invention.
FIG. 4 is a schematic diagram of a nucleic acid testing cassette with an added pre-amplification reagent chamber according to the invention.

### DESCRIPTION OF THE EMBODIMENTS

The invention will be further described below in conjunction with the accompanying drawings and specific embodiments.

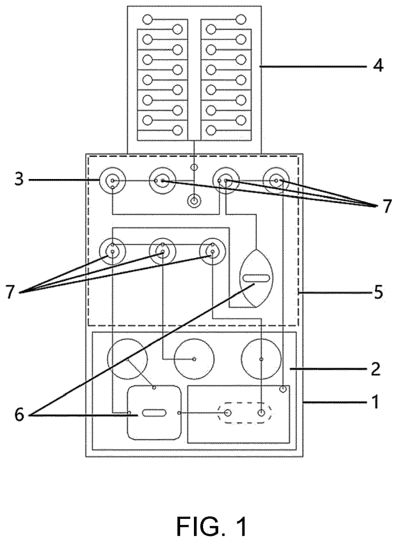

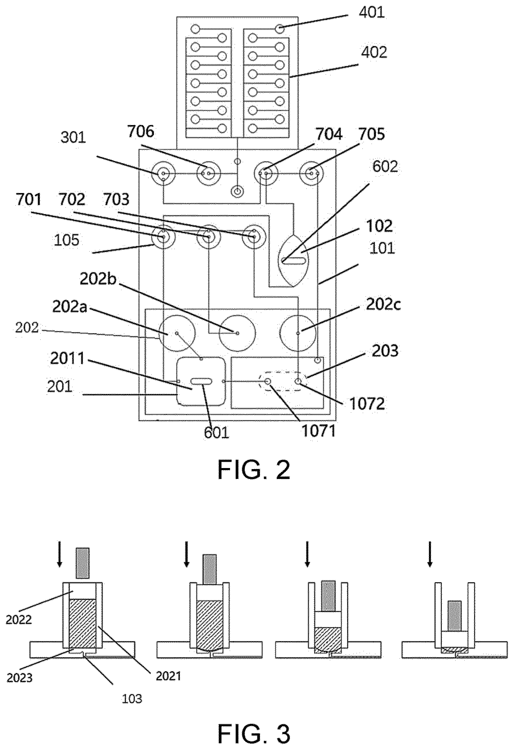

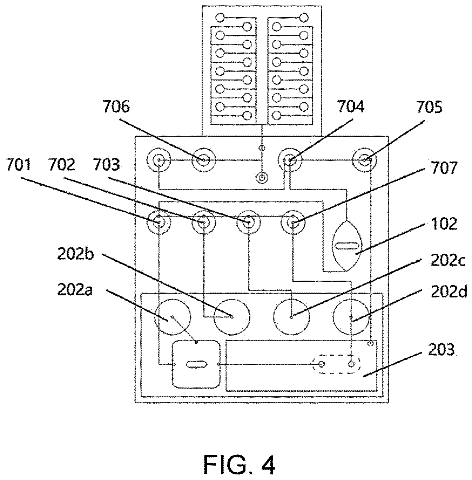

As shown in FIG. 1 to FIG. 4, the invention provides a nucleic acid testing cassette, which is an integrated cassette, including a substrate 1, a liquid storage component 2, a solid-reagent storage component 3, an amplification reaction region 4, a cover piece 5, rotor components 6, fluid isolating valves 7, and a casing. The components above are assembled and attached to form a sealed integrated cassette formed by a plurality of regions that are communicated with each other. The substrate 1 is connected to the amplification reaction region 4; the liquid storage component 2 and the solid-reagent storage component 3 are disposed on the substrate 1, respectively; the liquid storage component 2 is communicated with the solid-reagent storage component 3 through a micro flow channel; and the solid-reagent storage component 3 is communicated with the amplification reaction region 4 through a micro flow channel.

As shown in FIG. 1 to FIG. 4, the liquid storage component 2 includes a sample storage component 201, an extraction-reagent storage component 202, and a waste-liquid immobilization cavity 203; the substrate 1 is provided with a nucleic acid immobilization-extraction reaction pool 102; an output end of the sample storage component 201 and an output end of the extraction-reagent storage component 202 are communicated with an input end of the nucleic acid immobilization-extraction reaction pool 102 through a micro flow channel, respectively; and an output end of the nucleic acid immobilization-extraction reaction pool 102 is communicated with the solid-reagent storage component 3 and the waste-liquid immobilization cavity 203 through a micro flow channel, respectively.

As shown in FIG. 1 to FIG. 4, the solid-reagent storage component includes a storage cartridge 301 and a connecting structure; and the immobilization-reagent storage component 3 contains a reagent for nucleic acid amplification, such as dNTP or Taq polymerase, which is prestored in the storage cartridge 301 in the form of dry powder.

As shown in FIG. 1 to FIG. 4, the extraction-reagent storage component 202 includes a plurality of separate chambers, each of which includes a tube wall 2021, a plunger 2022, and a diaphragm 2023; the plungers 2022 are disposed above the tube walls 2021; the diaphragms 2023 are disposed below the tube walls 2021; the tube walls 2021, the plungers 2022, and the diaphragms 2023 enclose a liquid-reagent storage cavity; the substrate 1 is provided with sharp protrusions 103 in one-to-one correspondence to the chambers; and the sharp protrusions 103 are disposed below the diaphragms 2025 to pierce the diaphragms 2025 for releasing a liquid reagent within the liquid-reagent storage cavity.

As shown in FIG. 1 to FIG. 4, the chambers at least include a first chamber 202a storing a binding solution, a second chamber 202b storing a rinsing solution, and a third chamber 202c storing an eluent; the first chamber 202a is communicated with the sample storage component 201 through a micro flow channel; a micro flow channel between the sample storage component 201 and the nucleic acid immobilization-extraction reaction pool 102 is provided with a first fluid isolating valve 701; a micro flow channel between the second chamber 202b and the nucleic acid immobilization-extraction reaction pool 102 is provided with a second fluid isolating valve 702; a micro flow channel between the third chamber 202c and the nucleic acid immobilization-extraction reaction pool 102 is provided with a third fluid isolating valve 703; a micro flow channel between the nucleic acid immobilization-extraction reaction pool 102 and the solid-reagent storage component 3 is provided with a fourth fluid isolating valve 704; a micro flow channel between the nucleic acid immobilization-extraction reaction pool 102 and the waste-liquid immobilization cavity 203 is provided with a fifth fluid isolating valve 705; and a micro flow channel between the solid-reagent storage component 3 and the amplification reaction region 4 is provided with a sixth fluid isolating valve 706.

As shown in FIG. 1 to FIG. 4, the chambers further include a preamplification reagent chamber 202d storing a preamplification reagent; the preamplification reagent chamber 202d is communicated with the nucleic acid immobilization-extraction reaction pool 102 through a micro flow channel, on which a seventh fluid isolating valve 707 is disposed. According to a testing process, the preamplification reagent chamber 202d may further be added to prestore another necessary liquid reagent, for example, a nucleic acid amplification reagent, including a target probe, a specific primer, dNTP, Taq polymerase, a reaction buffer or the like, for use in first-step amplification of nested amplification.

As shown in FIG. 1 to FIG. 4, the substrate 3 is provided with a plurality of grooves 101 that are communicated with each other; and the cover piece 5 is attached to the substrate 1, and encloses the grooves 101 to form micro flow channels. After the cover piece 5 is attached to both sides of the substrate 1, a plurality of enclosed pipes and cavities that are communicated with each other are formed. The substrate 1 is provided with a plurality of valve seat structures 105, on which the plurality of isolating valves 7 are installed.

As shown in FIG. 1 to FIG. 4, the waste-liquid immobilization cavity 203 is provided with first and second air passage interfaces 1071 and 1072 that may be independently controlled to be opened or closed; the sample storage component 201 is communicated with the first air passage interface 1071; and the third chamber 202c or the preamplification reagent chamber 202d is communicated with the second air passage interface 1072.

As shown in FIG. 1 to FIG. 4, the rotor components 6 include a first magnetic rotor 601 and a second magnetic rotor 602; the sample storage component 201 is internally provided with the first magnetic rotor 601; the nucleic acid immobilization-extraction reaction pool 102 is internally provided with the second magnetic rotor 602; a first magnet rotating mechanism for driving the first magnetic rotor 601 to rotate is installed on the sample storage component 201; a second magnet rotating mechanism for driving the second magnetic rotor 602 to rotate is installed on the nucleic acid immobilization-extraction reaction pool 102; the first magnetic rotor 601 is a mixing-pool rotor, and the second magnetic rotor 602 is an immobilization-extraction reaction pool rotor; a first heating module is installed on the nucleic acid immobilization-extraction reaction pool 102; and a second heating module is installed on the amplification reaction region 4.

As shown in FIG. 1 to FIG. 4, the sample storage component 201 mainly consists of a cavity 2011 and a cover; and the cavity 2011 of the sample storage component 201 internally pre-stores nucleic-acid-capturing magnetic beads required for use in a nucleic acid extraction process.

As shown in FIG. 1 to FIG. 4, the waste-liquid immobilization cavity 203 contains a set of porous water-absorbing material, such as sponge and/or water absorbing paper, etc., and is provided with a waste-liquid outlet.

As shown in FIG. 1 to FIG. 4, the amplification reaction region 4 mainly consists of a plurality of reaction wells 401 and pipes 402 connecting the reaction wells 401; the amplification reaction region 4 is connected to the substrate 1 through a connecting piece; and a fluorescence-imaging processing module is installed above the amplification reaction region 4.

As shown in FIG. 1 to FIG. 4, the substrate 1 is attached to the liquid storage component 2, the solid-reagent storage component 3, and the cover piece 5 together in one or more of the manners such as buckling, gluing and hot pressing.

As shown in FIG. 1 to FIG. 4, the reaction wells 401 in the amplification reaction region 4 prestore target probes or primers for use in nucleic acid amplification in a dry form.

As shown in FIG. 1 to FIG. 4, main bodies of the substrate 1, the liquid storage component 2, the solid-reagent storage component 3, the amplification reaction region 4, and the cover piece 5 are made of high-molecular polymers, which may be one or more of polycarbonate, polymethyl methacrylate, cycloolefin copolymer, polypropylene, and polyethylene glycol terephthalate.

As shown in FIG. 1 to FIG. 4, the fluid isolating valves 7 are made of an elastic material with good airtightness, which may be one or more of natural rubber, silica gel, nitrile rubber, butyl rubber, fluororubber, and ethylene propylene rubber.

As shown in FIG. 1 to FIG. 4, the casing is made of a high-molecular polymer, which may be one or more of polycarbonate, polymethyl methacrylate, cycloolefin copolymer, polypropylene, polyethylene glycol terephthalate, and acrylonitrile-butadiene-styrene copolymer.

Embodiment 1 for use of the nucleic acid testing cassette according to the invention is as follows.

### Embodiment 1 - Integrated Extraction and Amplification

1. The following describes the implementation of the integrated cassette for automated nucleic acid extraction and testing as described in the invention.

2. A liquid sample (such as saliva, liquefied sputum, blood, and a swab rinsing solution) was added to the cavity 2011 of the sample storage component 201; then the cover was closed (not shown in the drawings); and the cassette was placed into a matched external instrument to start the automated nucleic acid extraction and testing.

3. The external instrument included at least 2 air passage interfaces (including the first air passage interface 1071 and the second air passage interface 1072) that might be independently controlled to be opened or closed, 10 compression levers, 2 local heating modules, 2 rotating mechanisms (including the first magnet rotating mechanism and the second magnet rotating mechanism) including magnets, and 1 fluorescence-imaging processing module. Each of the fluid isolating valves 7 was correspondingly provided with one compression lever for controlling the opening/closing of the fluid isolating valve 7; and the plunger 2022 in each of the chambers was correspondingly provided with one compression lever for controlling the compression of the plunger 2022.

4. When the integrated cassette already containing the sample to be tested was placed in the instrument, the plurality of compression levers closed the first fluid isolating valve 701, the second fluid isolating vale 702, the third fluid isolating valve 703, and the fourth fluid isolating valve 704 respectively; the first air passage interface 1071 was closed; and here, an independent closed space was formed in the cavity 2011. The first magnet rotating mechanism, below the cavity 2011, on the external instrument was started, such that the sample could be fully mixed and reacted with the embedded reagents. This step was intended to release the nucleic acid to be tested from the sample.

5. After the mixing was completed, the air passage interface 1071 was opened such that the compression lever on the second chamber 202a was lowered to push the plunger 2022 to press the diaphragm 2023, which expanded and deformed and was pierced by the sharp protrusion 103 on the substrate 1, thereby releasing a nucleic acid binding solution within the second chamber 202a.

6. While the first magnet rotating mechanism kept moving, the first magnetic rotor 601 fully mixed the released nucleic acid with the magnetic beads and the binding solution; then the first fluid isolating valve 701 was opened; and meanwhile, an air source provides a positive pressure into the cavity 2011 through the first air passage interface 1071, thereby pushing the above mixed solution to flow in an order of "the cavity 2011, the first fluid isolating valve 701 (opened), the nucleic acid immobilization-extraction reaction pool 102, the fourth fluid isolating valve 704 (closed), the fifth fluid isolating valve 705 (opened), and the waste-liquid immobilization cavity 203".

7. During this process, when magnetic-bead particles flowed to the nucleic acid immobilization-extraction reaction pool 102 along with the liquid, the second magnet rotating mechanism below the nucleic acid immobilization-extraction reaction pool 102 kept still; and under the action of a magnetic force, the magnetic-bead particles were immobilized in the nucleic acid immobilization-extraction reaction pool 102, and the liquid finally entered the waste-liquid immobilization cavity 203.

8. Then, the compression levers were adjusted in position, the first fluid isolating valve 701 was closed, and the second fluid isolating valve 702 was opened. Meanwhile, the compression lever on the second chamber 202b was lowered to push the plunger 2022 to press the diaphragm 2023, which expanded and deformed and was pierced by the sharp protrusion 103 on the substrate 1, thereby releasing a nucleic acid rinsing solution from the second chamber 202b. It should be specially noted that, during this process, the compression lever could release the liquid in the chamber by one compression, or release the liquid in batches at a fixed amount by controlling the lowering height, thereby performing rinsing more than once.

9. The nucleic acid rinsing solution released from the second chamber 202b entered the nucleic acid immobilization-extraction reaction pool 102 through the second fluid isolating valve 702, where the nucleic acid immobilization-extraction reaction pool 102 was a cavity with a low depth-to-width ratio and had a projection shape that might be round, rhombic, olivary, gourd-shaped or the like, and the foregoing various shapes guaranteed that the liquid might fully fill the cavity. After the nucleic acid immobilization-extraction reaction pool 102 was fully filled with the rinsing solution, the second magnet rotating mechanism below the nucleic acid immobilization-extraction reaction pool 102 started moving to drive the second magnetic rotor 602 to fully mix the magnetic beads immobilized in the cavity in the preceding step with the rinsing solution.

10. Then, the second magnet rotating mechanism stopped moving, and under the action of the magnetic force, the magnetic-bead particles suspending in the cavity after standing for a period of time were re-immobilized in the nucleic acid immobilization-extraction reaction pool 102.

11. After the immobilization of the magnetic beads was completed, the second air passage interface 1072 and the third fluid isolating valve 703 were opened; and under the action of an external air source, the rinsing solution within the nucleic acid immobilization-extraction reaction pool 102 was pushed into the waste-liquid immobilization cavity 203.

12. After the rinsing step was completed, the third fluid isolating valve 703 was kept opened, and the compression lever on the third chamber 203 was lowered to push the plunger 2032 to press the diaphragm 2033, which expanded and deformed and was pierced by the sharp protrusion 103 on the substrate 1, thereby releasing a nucleic acid eluent within the third chamber 202c.

13. The nucleic acid eluent released from the third chamber 202c entered the nucleic acid immobilization-extraction reaction pool 102 through the third fluid isolating valve 703. After the nucleic acid immobilization-extraction reaction pool 102 was fully filled with the eluent, the second magnet rotating mechanism below the nucleic acid immobilization-extraction reaction pool 102 started moving to drive the second magnetic rotor 602 to fully mix the magnetic beads immobilized in the cavity in the preceding step with the eluent; and meanwhile, the first heating module disposed below the nucleic acid immobilization-extraction reaction pool 102 was started. For "mixing plus heating" , a better effect was achieved by controlling the temperature to be 50-80°C and the time to be 180-600 s.

14. After the above operations were completed, the fourth fluid isolating valve 704 was opened, and the fifth fluid isolating valve 705 was closed; and under the action of the external air source, the eluent entered the storage cartridge 301 of the solid-reagent storage component 3 from the nucleic acid immobilization-extraction reaction pool 102, and was mixed with the reagent therein.

15. The compression lever above the storage cartridge 301 was lowered to push the mixed reagent to enter the reaction wells 401 of the amplification reaction region 4.

16. Then, by lowering the compression lever to close the sixth fluid isolating valve 706, each of the reaction wells 401 was isolated from the outside; the second heating module below the amplification reaction region 4 was opened; and the fluorescence-imaging processing module above the amplification reaction region 4 was opened. While the reaction temperature was controlled, a nucleic acid amplification reaction started occurred in each of the independent reaction wells; and the fluorescence-imaging processing module tested a target gene based on a light signal of each of the reaction wells.

### Embodiment 2 - Integrated Nucleic Acid Extraction and Nested Amplification

1. The following describes another implementation of the integrated cassette for automated nucleic acid extraction and testing as described in the invention.

2. In this embodiment, the operations in a first stage were substantially the same as Steps 1-13 in Embodiment 1. That is, the nucleic acid eluent in the third chamber 202c was injected into the nucleic acid immobilization-extraction reaction pool 102, but by controlling a lowering distance of a corresponding plunger 2032, the eluent was not allowed to fully fill the nucleic acid immobilization and extraction reaction pool 102.

3. After the nucleic acid elution was completed, the first fluid isolating valve 701, the second fluid isolating valve 702, the third fluid isolating valve 703, the fourth fluid isolating valve 704, the fifth fluid isolating valve 705, and the seventh fluid isolating valve 707 were kept opened; the plunger 2032 above the preamplification reagent chamber 202d was pressed down to release a certain amount of preamplification reagent into the nucleic acid immobilization-extraction reaction pool 102; the second magnet rotating mechanism below the nucleic acid immobilization-extraction reaction pool 102 moved to drive the second magnetic rotor 602 to fully mix the nucleic acid eluted in the preceding step with the preamplification reagent, where an optimal effect might be achieved when the mixing time was 5-60 s.

4. After the mixing was completed, the first fluid isolating valve 701, the second fluid isolating valve 702, the third fluid isolating valve 703, the fourth fluid isolating valve 704, the fifth fluid isolating valve 705, and the seventh fluid isolating valve 707 were closed; and the first heating module below the nucleic acid immobilization-extraction reaction pool 102 was started to perform the preamplification reaction of the nucleic acid. The amplification reaction might be isothermal nucleic acid amplification, or variable-temperature nucleic acid amplification.

5. After the preamplification was completed, the fifth fluid isolating valve 705 and the seventh fluid isolating valve 707 were opened; a flow rate of the external air source was controlled to allow part of amplification products to enter the waste-liquid immobilization cavity 203 through the fourth fluid isolating valve 704 and the fifth fluid isolating valve 705. The rest of the amplification products might account for 1/10-1/100 of a total amount in the nucleic acid immobilization-extraction reaction pool 102, or might be another appropriate ratio required based on the actual amplification reaction

6. The third fluid isolating valve 703 was opened; the fourth fluid isolating valve 704 was closed; the plunger 2032 above the third chamber 202c was pressed down to release the nucleic acid eluent to enter and fully fill the nucleic acid immobilization-extraction reaction pool 102. Here, the nucleic acid eluent plays a role of diluting the preamplification products. The second magnet rotating mechanism below the nucleic acid immobilization-extraction reaction pool 102 moved to drive the second magnetic rotor 602 to fully mix the preamplification products from the preceding step with the eluent, where an optimal effect might be achieved when the mixing time was 5-60 s.

7. After the operations described above were completed, the fourth fluid isolating valve 704 was opened; the fifth fluid isolating valve 705 was closed; and under the action of the external air source, the diluted preamplification products entered the storage cartridge 301 of the solid-reagent storage component 3 from the nucleic acid immobilization-extraction reaction pool 102, and were mixed with the reagent therein.

8. Subsequent operations were substantially the same as Steps 15-16 in Embodiment 1.

The nucleic acid testing cassette according to the invention has the following advantages:

1. all the reagents, including a liquid reagent and a dry-powder reagent, required for nucleic acid extraction and amplification can be internally disposed on an integrated cassette (i.e., a chip), and a user only needs to add a sample, such that the nucleic acid testing cassette is extremely easy to operate and truly suitable for primary users to use;

2. the nucleic acid testing cassette is applicable to nested amplification, and the sensitivity is greatly improved; and

3. all the components are made of common materials by using common processes in the medical industry, such that the cost is greatly reduced.

The description above provides further detailed explanation of the invention in conjunction with the specific preferred embodiments, and it should not be deemed that the specific implementation of the invention is limited to these explanations. For those of ordinary skill in the art to which the invention belongs, several simple deductions or substitutions can also be made without departing from the conception of the invention, and should be construed as falling within the protection scope of the invention.

## Claims

1. A nucleic acid testing cassette, **characterized by** comprising a substrate, a liquid storage component, a solid-reagent storage component, and an amplification reaction region, wherein the substrate is connected to the amplification reaction region; the liquid storage component and the solid-reagent storage component are disposed on the substrate, respectively; the liquid storage component is communicated with the solid-reagent storage component through a micro flow channel; and the solid-reagent storage component is communicated with the amplification reaction region through a micro flow channel.

2. The nucleic acid testing cassette according to Claim 1, **characterized in that** the liquid storage component comprises a sample storage component, an extraction-reagent storage component, and a waste-liquid immobilization cavity; the substrate is provided with a nucleic acid immobilization-extraction reaction pool; an output end of the sample storage component and an output end of the extraction-reagent storage component are respectively communicated with an input end of the nucleic acid immobilization-extraction reaction pool through a micro flow channel, respectively; and an output end of the nucleic acid immobilization-extraction reaction pool is communicated with the solid-reagent storage component and the waste-liquid immobilization cavity through a micro flow channel, respectively.

3. The nucleic acid testing cassette according to Claim 2, **characterized in that** the extraction-reagent storage component comprises a plurality of separate chambers, each of the plurality of separate chambers comprises a tube wall, a plunger, and a diaphragm; the plungers are disposed above the tube walls; the diaphragms are disposed below the tube walls; the tube walls, the plungers, and the diaphragms enclose a liquid-reagent storage cavity; the substrate is provided with sharp protrusions in one-to-one correspondence to the chambers; and the sharp protrusions are disposed below the diaphragms.

4. The nucleic acid testing cassette according to Claim 3, **characterized in that** the chambers at least comprise a first chamber storing a binding solution, a second chamber storing a rinsing solution, and a third chamber storing an eluent; the first chamber is communicated with the sample storage component through a micro flow channel; a micro flow channel between the sample storage component and the nucleic acid immobilization-extraction reaction pool is provided with a first fluid isolating valve; a micro flow channel between the second chamber and the nucleic acid immobilization-extraction reaction pool is provided with a second fluid isolating valve; a micro flow channel between the third chamber and the nucleic acid immobilization-extraction reaction pool is provided with a third fluid isolating valve; a micro flow channel between the nucleic acid immobilization-extraction reaction pool and the solid-reagent storage component is provided with a fourth fluid isolating valve; a micro flow channel between the nucleic acid immobilization-extraction reaction pool and the waste-liquid immobilization cavity is provided with a fifth fluid isolating valve; and a micro flow channel between the solid-reagent storage component and the amplification reaction region is provided with a sixth fluid isolating valve.

5. The nucleic acid testing cassette according to Claim 4, **characterized in that** the chambers further comprise a preamplification reagent chamber storing a preamplification reagent; and the preamplification reagent chamber is communicated with the nucleic acid immobilization-extraction reaction pool through a micro flow channel, on which a seventh fluid isolating valve is disposed.

6. The nucleic acid testing cassette according to Claim 5, **characterized in that** the waste-liquid immobilization cavity is provided with first and second air passage interfaces that may be independently controlled to be opened or closed; the sample storage component is communicated with the first air passage interface; and the third chamber or the preamplification reagent chamber is communicated with the second air passage interface.

7. The nucleic acid testing cassette according to Claim 4, **characterized in that** the substrate is provided with a plurality of grooves that are communicated with each other; a cover piece is attached to the substrate, and encloses the grooves to form micro flow channels; and the substrate is provided with a plurality valve seat structures, on which the first fluid isolating valve, the second fluid isolating valve, the third fluid isolating valve, the fourth fluid isolating valve, the fifth fluid isolating valve, and the sixth fluid isolating valve are installed, respectively.

8. The nucleic acid testing cassette according to Claim 2, **characterized in that** the sample storage component is internally provided with a first magnetic rotor; the nucleic acid immobilization-extraction reaction pool is internally provided with a second magnetic rotor; a first magnet rotating mechanism for driving the first magnetic rotor to rotate is installed on the sample storage component; a second magnet rotating mechanism for driving the second magnetic rotor to rotate is installed on the nucleic acid immobilization-extraction reaction pool; a first heating module is installed on the nucleic acid immobilization-extraction reaction pool; and a second heating module is installed on the amplification reaction region.

9. The nucleic acid testing cassette according to Claim 2, **characterized in that** the sample storage component mainly consists of a cavity and a cover; the cavity of the sample storage component internally pre-stores nucleic-acid-capturing magnetic beads required for use in a nucleic acid extraction process; and a porous water-absorbing material is contained in the waste-liquid immobilization cavity.

10. The nucleic acid testing cassette according to Claim 1, **characterized in that** the amplification reaction region mainly consists of a plurality of reaction wells and pipes connecting the reaction wells; the amplification reaction region is connected to the substrate through a connecting piece; and a fluorescence-imaging processing module is installed above the amplification reaction region.
